# EUROPEAN PATENT SPECIFICATION

(11) **EP 1 083 952 B1**
(45) Date of publication and mention of the grant of the patent: **14.12.2005**
(21) Application number: 99930254.0
(22) Date of filing: 14.06.1999
(51) Int. Cl.: A61M 15/00, A61J 3/00, B05B 5/08

(54) **DEVICE FOR DELIVERY OF PHARMACEUTICALS AND DRUGS**
VORRICHTUNG ZUR ABGABE VON ARZNEIMITTELN
DISPOSITIF D'ADMINISTRATION DE SUBSTANCES PHARMACEUTIQUES ET DE MEDICAMENTS

(30) Priority: 12.06.1998 US 97104; 12.06.1998 US 97105; 12.06.1998 US 97106
(43) Date of publication of application: 21.03.2001
(73) Proprietor: Microdose Technologies Inc., Monmouth Jct., NJ 08852 (US)
(72) Inventor: ABRAMS, Andrew, L., Westport, CT 06880 (US); GUMASTE, Anand, V., Robbinsville, NJ 08691 (US); FLEMING, Scott, Ewing, NJ 08628 (US)
(74) Representative: Audier, Philippe André
(86) International application number: PCT/US1999/013420
(87) International publication number: WO 1999/064095

(56) References cited:
- WO-A-97/26934
- WO-A-98/20861
- GB-A- 2 262 452
- US-A- 4 349 531
- US-A- 5 284 133
- US-A- 5 331 953
- US-A- 5 363 842
- US-A- 5 551 416
- US-A- 5 655 523
- US-A- 5 699 649
- US-A- 5 714 007
- US-A- 5 735 263

## Description

The present invention relates generally to the field of metering, packaging and delivery of pharmaceuticals and drugs. Particular utility for the present invention is found in the area of facilitating metering and packaging of dry powder medications and/or inhalation of powdered medications, although other utilities are contemplated, including other medicament applications.

Certain diseases of the respiratory tract are known to respond to treatment by the direct application of therapeutic agents. As these agents are most readily available in dry powdered form, their application is most conveniently accomplished by inhaling the powdered material through the nose or mouth. This powdered form results in the better utilization of the medicament in that the drug is deposited exactly at the site desired and where its action may be required; hence, very minute doses of the drug are often equally as efficacious as larger doses administered by other means, with a consequent marked reduction in the incidence of undesired side effects and medicament cost. Alternatively, the drug in this form may be used for treatment of diseases other than those of the respiratory system. When the drug is deposited on the very large surface areas of the lungs, it may be very rapidly absorbed into the blood stream; hence, this method of application may take the place of administration by injection, tablet, or other conventional means.

It is the opinion of the pharmaceutical industry that the bioavailability of the drug is optimum when the drug particles delivered to the respiratory tract are between 1 to 5 microns in size. When the drug particles need to be in this size range the dry powder delivery system needs to address a number of issues:
(1) Small size particles develop an electrostatic charge on themselves during manufacturing and storage. This causes the particles to agglomerate or aggregate, resulting in clusters of particles which have an effective size greater than 5 microns. The probability of these large clusters making it to the deep lungs then decreases. This in turn results in a lower percentage of the packaged drug being available to the patient for absorption.
(2) The amount of active drug that needs to be delivered to the patient may be of the order of 10s of micrograms. For example, albuterol, in the case of a drug used in asthma, this is usually 25 to 50 micrograms. Current manufacturing equipment can effectively deliver aliquots of drugs in milligram dose range with acceptable accuracy. So the standard practice is to mix the active drug with a filler or bulking agent such as lactose. This additive also makes the drug "easy to flow". This filler is also called a carrier since the drug particles also stick to these particles through electrostatic or chemical bonds. These carrier particles are very much larger than the drug particles in size. The ability of the dry powder inhaler to separate drug from the carrier is an important performance parameter in the effectiveness of the design.
(3) Active drug particles with sizes greater than 5 microns will be deposited either in the mouth or throat. This introduces another level of uncertainty since the bioavailability and absorption of the drug in these locations is different from the lungs. Dry powder inhalers need to minimize the drug deposited in these locations to reduce the uncertainty associated with the bioavailability of the drug.

Prior art dry powder inhalers (DPIs) usually have a means for introducing the drug (active drug plus carrier) into a high velocity air stream. The high velocity air-stream is used as the primary mechanism for breaking up the cluster of micronized particles or separating the drug particles from the carrier. Several inhalation devices useful for dispensing this powder form of medicament are known in the prior art. For example, in U.S. Patent Nos. 3,507,277; 3,518.992; 3,635,219; 3,795,244; and 3,807,400, inhalation devices are disclosed having means for piercing of a capsule containing a powdered medicament, which upon inhalation is drawn out of the pierced capsule and into the user's mouth. Several of these patents disclose propeller means, which upon inhalation aid in dispensing the powder out of the capsule, so that it is not necessary to rely solely on the inhaled air to suction powder from the capsule. For example, in U.S. Patent No. 2,517,482. a device is disclosed having a powder containing capsule placed in a lower chamber before inhalation, where it is pierced by manual depression or a piercing pin by the user. After piercing, inhalation is begun and the capsule is drawn into an upper chamber of the device where it moves about in all directions to cause a dispensing of powder through the pierced holes and into the inhaled air stream. U.S. Patent No. 3,831,606 discloses an inhalation device having multiple piercing pins, propeller means, and a self-contained power source for operating the propeller means via external manual manipulation, so that upon inhalation the propeller means aids in dispensing the powder into the stream of inhaled air. See also U.S. Patent No. 5,458,135.

These prior art devices present several problems and possess several disadvantages which are remedied by the inhalation devices of the present invention. For instance, these prior art devices require that the user exert considerable effort in inhalation to effect dispensing or withdrawal of powder from a pierced capsule into the inhaled air stream. With these prior art devices, suction of powder through the pierced holes in the capsule caused by inhalation generally does not withdraw all or even most of the powder out of the capsule, thus causing a waste of the medicament. Also, such prior art devices result in uncontrolled amounts or clumps of powdered material being inhaled into the user's mouth, rather than a constant inhalation of controlled amounts of finely dispersed powder.

The above description of the prior art is taken largely from U.S. Pat. No. 3,948,264 to Wilke et al, who disclose a device for facilitating inhalation of a powdered medication that includes a body portion having primary and secondary air inlet channels and an outlet channel. The secondary inlet channel provides an enclosure for a capsule containing the powdered medication and the outlet channel is formed as a mouthpiece protruding from the body. A capsule piercing structure is provided, which upon rotation puts one or more holes in the capsule so that upon vibration of the capsule by an electro-mechanical vibrator, the powdered drug may be released from the capsule. The piercing means disclosed in Wilke et al includes three radially mounted, spring-biased piercing needles mounted in a trochoidal chamber. Upon hand rotation of the chamber, simultaneous inward radial motion of the needles pierces the capsule. Further rotation of the chamber allows the needles to be retracted by their spring mountings to their original positions to withdraw the needles from the capsule. The electromechanical vibrator includes, at its innermost end, a vibrating plunger rod which projects into the intersection of the inlet channel and the outlet channel. Connected to the plunger rod is a mechanical solenoid buzzer for energizing the rod to vibrate. The buzzer is powered by a high energy electric cell and is activated by an external button switch. According to Wilke et al, upon inhalation through outlet channel 3 and concurrent pressing of switch 10d to activate the electromechanical vibrating means 10, air is sucked through inlet channels 4 and 12 and the air stream through the secondary inlet channel 4 raises the capsule up against the vibrating plunger rod 10a. The capsule is thus vibrated rapidly with powder being fluidized and dispensed from the pierced holes therein. (This technique is commonly used in manufacturing for dispensing powder through a hopper where the hopper is vibrated to fluidize the powder and move it through the hopper outlet. The pierced holes in the capsule represent the hopper outlet.) The air stream through inlet channel 4 and 12 aids in withdrawal of powder from the capsule and carries this powder through the outlet channel 3 to the mouth of the user." (Wilke et al, column 3, lines 45-55). Wilke et al further discloses that the electromechanical vibrator means may be placed at a right angle to the inlet chamber and that the amplitude and frequency of vibration may be altered to regulate dispensing characteristics of the inhaler.

Thus, as noted above, the vibrator in Wilke et al's disclosed inhaler is an electromechanical device consisting of a rod driven by a solenoid buzzer. (This electromechanical means may be a motor driving a cam [Col. 4, Line 40]). A disadvantage of the inhaler implementation as disclosed by Wilke is the relatively large mechanical movement required of the rod to effectively vibrate the capsule. The large movement of the rod, usually around 100s of microns, is necessary due to the elasticity of the capsule walls and inertia of the drug and capsule.

Moreover, solenoid buzzers typically have operating frequencies less than 5 Khz. This operating frequency tends to be noisy and therefore is not desirable when incorporated into a dry powder inhaler from a patient's perspective. A further disadvantage of the electrochemical actuators of Wilke is the requirement for a high energy source (Wilke et al. Col. 3, line 38), thus requiring a large battery source or frequent changes of the battery pack for portable units. Both these features are not desirable from a patient safety and "ease of use" standpoint.

The inhaler of Wilke et al is primarily intended to reduce the amount of powder left behind in the capsule relative to other inhalers cited in the patent disclosure. (Wilke et al, Col. 4, lines 59-68, Col. 5, lines 1-48). However, Wilke et al does not address the need to deaggregate the powder into particle sizes or groups less than 6 microns in size as is required for effective delivery of the medication to the lungs; rather Wilke et al, like the prior art inhalers continues to rely on the air stream velocity to deaggregate the powder ejected into the air stream, into particle sizes suitable for delivery to the lungs.

Another prior art inhalation device is disclosed in Bums et al U.S. Patent No. 5,284,133. In this device, a liquid medication is atomized by an ultrasonic device such as a piezo element (Bums et al, Col. 10, lines 36-51). A stream of air, usually at a high velocity, or a propellant then carries the atomized particles to the patient. The energy required to atomize the liquid medication in the nebulizer is prohibitively high, making this approach for the delivery of drugs to the lungs only feasible as a desk top unit. The high voltage requirements to drive the piezo, to produce the necessary mechanical displacements, also severely effects the weight and size of the device. It is also not obvious that the nebulizer operating principles can be applied to the dry powder inhalers for delivery or powder medication to the lungs.

Another prior art inhalation device is disclosed in US-A-5 331 953.In this device, a microphone and an electronic unit are used to record the time each dosage is taken through the inhaler. They are passive and sense and create a record of usage.

The prior art devices therefore have a number of disadvantages which makes them less than desirable for the delivery of dry powder to the lungs. Some of these disadvantages are:
- The performance of the prior art inhalers depends on the flowrate generated by the user. Lower flowrate does not result in the powder being totally deaggregated and hence adversely affects the dose delivered to the patient.
- Inconsistency in the bioavailability of the drugs from dose-to-dose because of lack of consistency in the deaggregation process.
- Large energy requirements for driving the electromechanical based inhalers which increases the size of the devices making them unsuitable for portable use.

In our prior U.S. Patent No. 5,694,920, issued December 9,1997, we provide an inhaler that utilizes vibration to facilitate suspension of powder into a gas that overcomes the aforesaid and other disadvantages and drawbacks of the above prior art. More particularly, the inhaler of our aforesaid patent includes a piezoelectric vibrator for vibrating the powder. A controller is provided for controlling supply (i.e., amplitude and/or frequency) of actuating electricity to the vibrator so as to cause vibration of the powder that is adapted to optimally suspend at least a portion of the powder into the gas. As described in our aforesaid patent, the controller may include a user-actuable control for permitting the user to select the vibration frequencies and/or amplitudes for optimally suspending in the gas the type of powder currently being used in the inhaler. The user-actuable control is pre-calibrated with the controller to cause the controller to adjust the frequency and /or amplitude of actuating electricity supplied to the vibrator to be that necessary for vibrating the type of powder selected by the user-actuable control in such a way as to optimally suspend at least a portion of the powder into the gas. The user-actuable control may include selection gradations in terms of the average size of the powder particles to be suspended in the gas, and/or in terms of desired vibration frequencies and amplitudes. Vibration frequency would be adjusted to at least about 12 KHz, in order to optimally suspend such commonly used powdered medications in the gas. Of course, vibration frequency and amplitude may be adjusted to optimize suspension of the powdered medication being used.

An electrostatic field that is established across the air stream, whereby by controlling the strength of the electrostatic field primarily only particle sizes of interest are introduced into the air stream, while larger size particles are left behind in the container. This reduces the inconsistency associated with the bioavailability of the drug because of the large particles being deposited into the mouth or throat as is common with devices described in prior art.

The present invention provides an improvement over prior art inhalation devices such as described in our aforesaid U.S. Patent No. 5,694,920. The invention is defined in the appended claim 1. In one embodiment of the invention, the inhaler contains two or more vibrator means or piezoelectric elements so that different drugs, i.e. of different particle size, may be delivered from the same inhaler.

In yet another embodiment of the invention, the piezoelectric elements are switched between two or more set frequencies, or frequencies swept so as to avoid potentially setting up standing waves in the powder.

In yet another embodiment of the invention, the inhaler includes electronic circuitry for recording and/or controlling one or more functions such as dose counting, patient compliance monitoring, and patient compliance reminders. Also, the inhaler may be programmed according to a delivery protocol, i.e. to alter the quantity of drug delivered over time. If desired, the inhaler also may include an environmental sensor and knockout control, for example, to deactivate the inhaler in the event it is inadvertently exposed to too high a temperature, a clock to deactivate the inhaler in the event its shelf life is exceeded and/or a security/safety lock-out.

More particularly, the present invention provides an air flow sensor for controlling various components of an inhalation device. Included in the preferred embodiment is an acoustic controller, the acoustic controller including an acoustic element to sense air flow around the element and for producing signals representative of a frequency and amplitude of the air flow, the signals being used to control (e.g., activated, deactivate, apply incremental voltage, etc.) certain components of the inhalation device.

Preferably, acoustic element is a microphone element or pressure transducer positioned within the air passage of an inhalation device, (e.g., a dry powder inhaler) that produces signals in response to the inhalation air flow, these signals are used to control certain components of the inhaler, e.g., a high frequency vibrator, an electrostatic plate, timer, counter, etc. Also preferably, these signals are used to activate/control certain components of the inhalation device to maximize the inhalation effectiveness to obtain maximum patient benefit from medicament.

Thus, the present invention provides a fully automated inhalation device, that is breath activated, that permits optimal utilization of the particular medication. For example, acoustic signals can be used to trigger the high frequency vibrator and electrostatic plate only when the patient has achieved optimum (e.g., maximum) inhalation effort, thereby ensuring that the full (proper) dosage of medicament properly enter the patient's respiratory system. Alternatively, these signals (breath-activated signals) can be used to progressively apply increasing power to, or, sequentially activate/deactivate the various components of the inhalation device to achieve optimal inhalation dosage.

Still other features and advantages of the present invention may be seen from the following detailed description, taken in connection with the attached drawings, wherein like numerals depict like parts, and wherein:
Figure 1 is a perspective view of an inhaler of the prior art;
Figure 2 is a rear plane view of the inhaler shown in Figure 1;
Figure 3 is a longitudinal cross-sectional schematic view of the inhaler of Figure 1;
Figure 4 is a functional block diagram of the vibration control system of one embodiment of Figure 1;
Figure 5 is a functional block diagram of the vibration control system of another embodiment of the invention;
Figures 6-10 are function block diagrams of the vibration control system in accordance with still yet other embodiments of the invention; and
Figure 11 is a view, similar to Figure 3 of yet another embodiment of the invention.
Figure 12 is a cross-sectional view of a typical inhalation device and the acoustic controller of the present invention;
Figure 13 is an expanded cross-sectional view of Figure 12;
Figure 14 is a functional block diagram of a preferred embodiment of the acoustic controller of the present invention;
Figure 15 shows a schematic representation of the attraction of negatively charged powder particles to a support having a positive charge on the surface thereof.

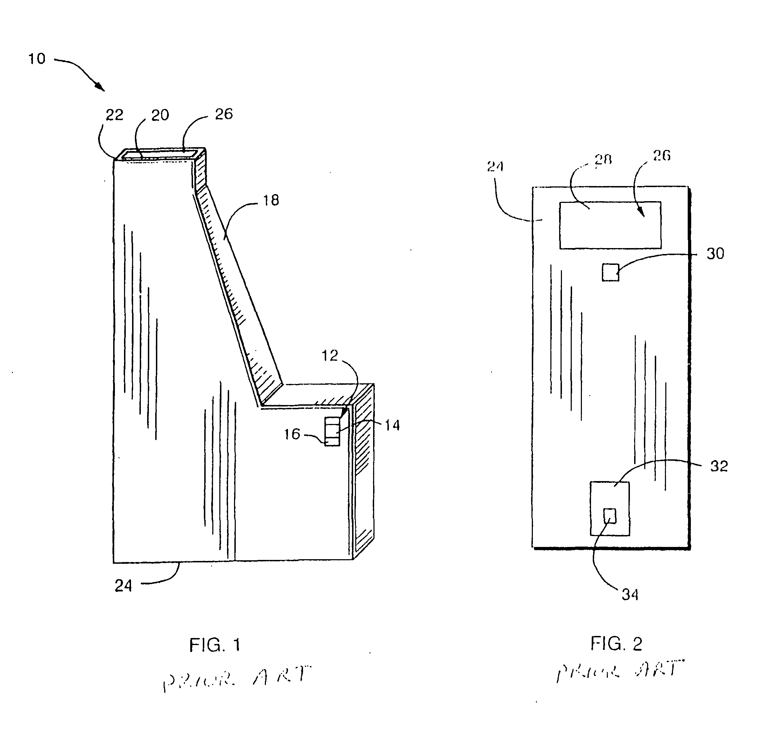

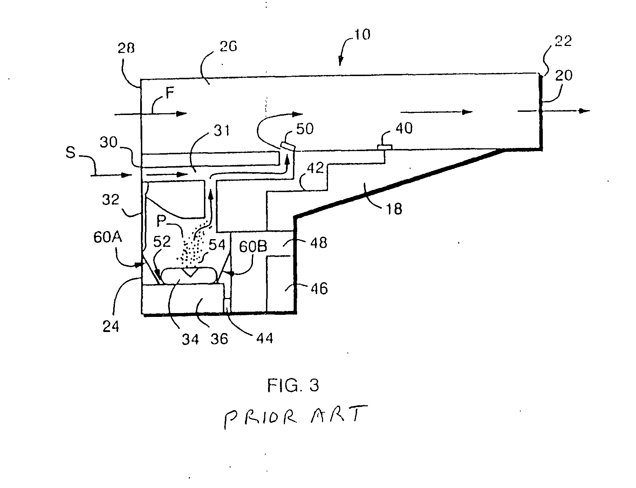

Figures 1-3 illustrate an embodiment 10 of inhaler made in accordance with our aforesaid U.S. Patent No. 5,694,920. Inhaler 10 includes a hard plastic or metal housing 18 having a generally L-shaped longitudinal cross-section. Housing 18 includes four air flow openings 20, 28, 30, and 32. Inhaler 10 includes a main air flow passage 26 which extends the length of the housing 18 from the front 22 (at opening 20) to the rear 24 thereof (at opening 28) and has a generally square-shaped transverse cross-section, so as to permit air flow therethrough (denoted by arrow F in Figure 1).

Secondary air conduit 31 is generally L-shaped and runs longitudinally from opening 30 in the rear 24 surface of the housing 18 to main passage 26. One-way flow valve 50 is mounted to the inner surface 33 of the main passage 26 via a conventional spring-biased hinge mechanism (not shown), which is adapted to cause the valve 50 to completely block air flow S through the conduit 31 to the main passage 26 when the pressure of the air flow F in the main passage 26 is below a predetermined threshold indicative of inhalation through the passage 26 by a user.

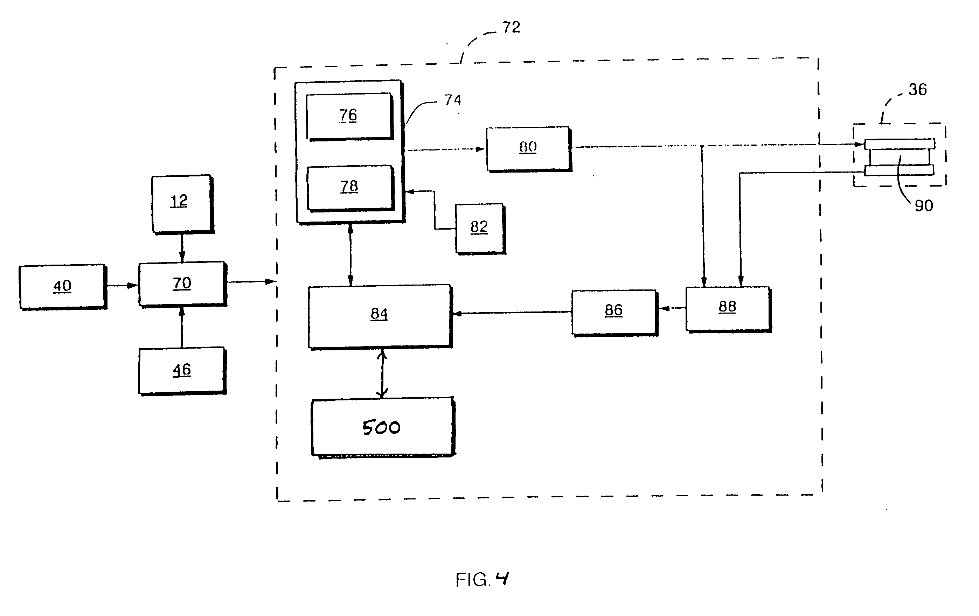

Powder dispensing chamber 54 is formed in housing 18 for holding a capsule 34 of powder medication to be inhaled. Housing 18 includes a moveable panel portion 32 in the rear 24 for permitting the capsule 34 to be introduced into the chamber 54 and placed on the seating 52 of vibration means 36 between guiding means 60A, 60B. Preferably, means 36 comprises a hard plastic or metallic protective shell 37 enclosing a piezoelectric vibrator 90. (Figure 4). Preferably, vibrator 90 is mechanically coupled through the shell 37 via a disk (not shown) to the drug cartridge 34 so as to permit maximum vibratory energy to be transmitted from the vibrator 90 through the shell 37 to the cartridge 34. Guiding means 60A, 60B includes two surfaces which slant downwardly toward the seating 52 so as to permit easy introduction and retention of the capsule on the seating 52 in the chamber 51. Removable panel 32 includes another air inlet 34 for permitting additional air flow S2 from the chamber 51 through conduit 61 into conduit 31 during inhalation by the user. Preferably, panel 32 and housing 18 include conventional mating mounting means (not shown) for permitting the panel 32 to be removably resecurable to the housing by the user between introduction of fresh (i.e., completely full) capsules and removal of spent (i.e., empty) capsules.

Inhaler 10 also includes a conventional miniature air stream velocity or pressure sensor 40 mounted on the inner surface of the conduit 26 so as to sense the speed and/or pressure of the air stream F. Preferably, sensor 40 comprises a conventional spring-loaded flapper-yield switch which generates electronic signals indicative of the speed and/or pressure of the air stream F in the conduit 26, and transmits those signals via electrical connection 42 to electronic control circuitry 48 contained in housing 18 for controlling actuation of the vibrator means based upon those signals.

Preferably, the control circuitry 48 is embodied as an application specific integrated circuit chip and/or some other type of very highly integrated circuit chip. Alternatively, control circuitry 48 may take the form of a microprocessor, or discrete electrical and electronic components. As will be described more fully below, the control circuitry 48 determines the amplitude and frequency of actuating power to be supplied from conventional power source 46 (e.g., one or more D.C. batteries) to the piezoelectric vibrator to thereby control vibration of the vibrator. The actuating power is supplied to the piezoelectric element 90 via electrical connection 44 between the vibrator and the circuitry 48.

Piezoelectric element 90 is made of a material that has a high-frequency, and preferably, ultrasonic resonant vibratory frequency (e.g., about 15 to 100 MHz), and is caused to vibrate with a particular frequency and amplitude depending upon the frequency and/or amplitude of excitation electricity applied to the piezoelectric element 90. Examples of materials that can be used to comprise the piezoelectric element 90 include quartz and polycrystalline ceramic materials (e.g., barium titanate and lead zirconate titanate). Advantageously, by vibrating the piezoelectric element 90 at ultrasonic frequencies, the noise associated with vibrating the piezoelectric element 90 at lower (i.e., non-ultrasonic) frequencies can be avoided.

Turning specifically to Figure 4, the various functional components and operation of the control circuitry 48 will now be described. As will be understood by those skilled in the art, although the functional components shown in Figure 4 are directed to an analog realization of the control circuitry 48, the components of Figure 4 could be appropriately modified to realize control circuitry 48 in a digital embodiment without departing from this embodiment 10 of the present invention.

Control circuitry 48 preferably includes actuation controller 70 and vibratory feedback control system 72. Actuation controller 70 comprises a conventional switching mechanism for permitting actuating power to be supplied from the power source 46 to the control system 72 depending upon the signals supplied to it from sensor 40 and the state or the power switch 12. In other words, controller 70 permits actuating power to be supplied from the source 46 to the system 72 when the sliding indicator bar 14 of switch 12 is set to the "ON" position in channel track 16 and the inhalation sensor 40 supplies signals to the controller 70 that indicate that the inhalation is occurring through the main passage 26. However, controller 70 does not permit actuating power to flow from the source to the system 72 when either the switch 12 is set to "OFF" or the signals supplied to the controller 70 from the sensor 40 indicate that inhalation is not taking place through the conduit 26.

When controller 70 first permits actuating power to be supplied from the source 46 to the feedback control system 72, the system 72 enters an initialization state wherein controllable means for supplying a predetermined frequency and amplitude of actuating electricity 74 is caused to generate control signals for causing conventional pump circuit 80 to generate an initial desired frequency and amplitude of actuating electricity based upon stored values thereof stored in the initialization memory means 82. Preferably, means 74 comprises conventional frequency sweep generator and frequency generator means 76 and 78, respectively. The signals generated by means 74 are then supplied to charge pump circuit 80 to cause circuit 80 to supply the piezoelectric element 90 with actuating electricity specified by the signals.

Preferably, the initial frequency and amplitude of actuating electricity supplied to the piezoelectric element 90 is pre-calibrated to cause the piezoelectric element 90 to vibrate at its resonance frequency when no powder cartridge or powder is placed on the means 36. As will be appreciated by those skilled in the art, maximum transfer of vibratory power from the piezoelectric element to the powder in the container 34 takes place when the piezoelectric element vibrates at its resonant frequency. It has been found that this results in maximum de-aggregation and suspension of the powder from the container 34 into the air to be inhaled by the user. However, when the container 36 or powder is placed on the vibrator means 36, the weight and volume of the powder container, and the weight, volume, and particular size of the powder to be suspended by the piezoelectric element can change the vibration characteristics of the piezoelectric element, and cause the piezoelectric element to vibrate at other than its resonant frequency. This can result in reduced vibratory energy transfer to the powder from the piezoelectric element, and thereby, lessen the efficiency of the piezoelectric element in de-aggregating and suspending the powder in the air inhaled by the user.

The feedback control system 72 overcomes this problem. In control system 72, after the initial frequency and amplitude of actuating electricity are supplied to the piezoelectric element 90, the frequency generating means 74 systematically generates control signals indicative of many different amplitudes and frequencies of electricity for being supplied to the piezoelectric element 90 by the circuit 80. As the generating means 74 "cycles through" the different frequencies and amplitudes, the instantaneous power transfer characteristics of the piezoelectric element 90 for each of these different frequencies and amplitudes are determined by the detector 88, which transmits this information to the peak power detector 86. Peak detector 86 analyzes the instantaneous power transfer characteristics of the piezoelectric element 90 and signals the sample and hold feedback controller 84 when the power transfer characteristics are at local maxima. The controller 84 correlates these local maxima with the frequencies and amplitudes commanded by the generator 74 to be supplied to the piezoelectric element 90.

After the frequency generator 74 has finished its sweep through the frequencies and amplitudes of power supplied to the piezoelectric element 90, the controller 84 causes the generator 74 to cycle through the frequencies and amplitudes of power that resulted in local maxima, and then determines which of these frequencies and amplitudes results in optimal power transfer characteristics through the piezoelectric element 90.

Completing the controller 72 is a clock 500 which is tripped when actuating electricity is first supplied to the piezoelectric element 90. Clock 500 includes a counter which prevents a second activation of the piezoelectric element for a preset period of time. Thus, overuse and overdosing by the patient are prevented.

In operation of embodiment 10, the drug-containing package 34 is punctured and inserted onto the surface 52 of vibrator 36 in chamber 51 in the manner described previously. The power switch is placed in the "ON" position and the user inhales air through the conduit 26, air flow F is generated through conduit 26. This causes one-way valve 50 to deflect to admit air flow S through opening 30 into conduit 26, and also causes air flow S2 through opening 34 and chamber 51 into conduit 26. The inhalation of air stream F is sensed by sensor 40 and is signaled to actuation controller 70, which causes power to be supplied to the controller 72. The controller 72 then adjusts the amplitude and frequency of actuating power supplied to the piezoelectric element until they are optimized for the best possible de-aggregation and suspension of the powder P from the capsule into the air stream F via air flows S and S2.

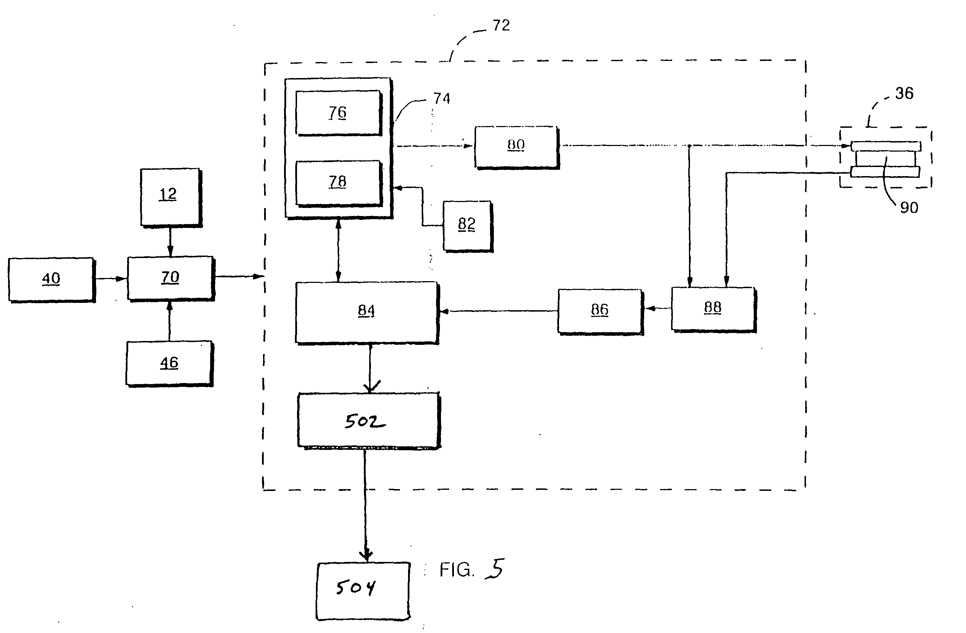

Figure 5 illustrates another embodiment of the invention. Figure 5 is similar to Figure 4, except the clock 500 is replaced with a counter 502 which counts the number of doses delivered bv the device. Counter 502 is connected to a display 504 which displays the number of doses delivered, or, optionally, the number of doses remaining.

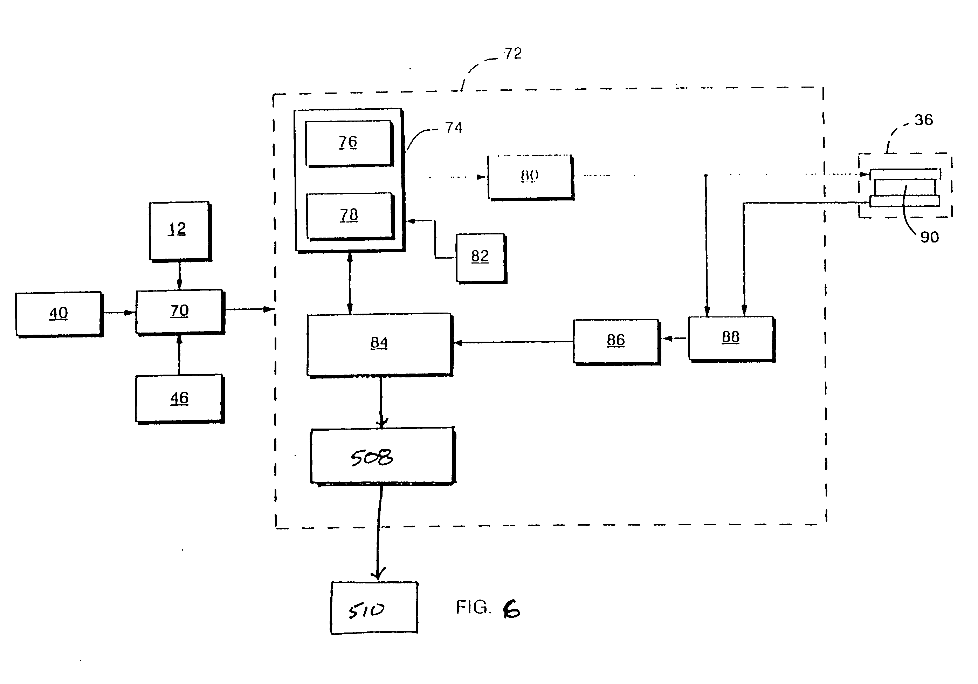

Figure 6 illustrates yet another embodiment of the invention. The Figure 6 embodiment is similar to the Figure 4 embodiment, except the clock 500 is replaced by an internal monitor which contains a record of inhaler use. Completing the Figure 6 embodiment is a hatch 510 through which a physician may access, read and/or download the data from monitor 508, whereby to determine patient compliance.

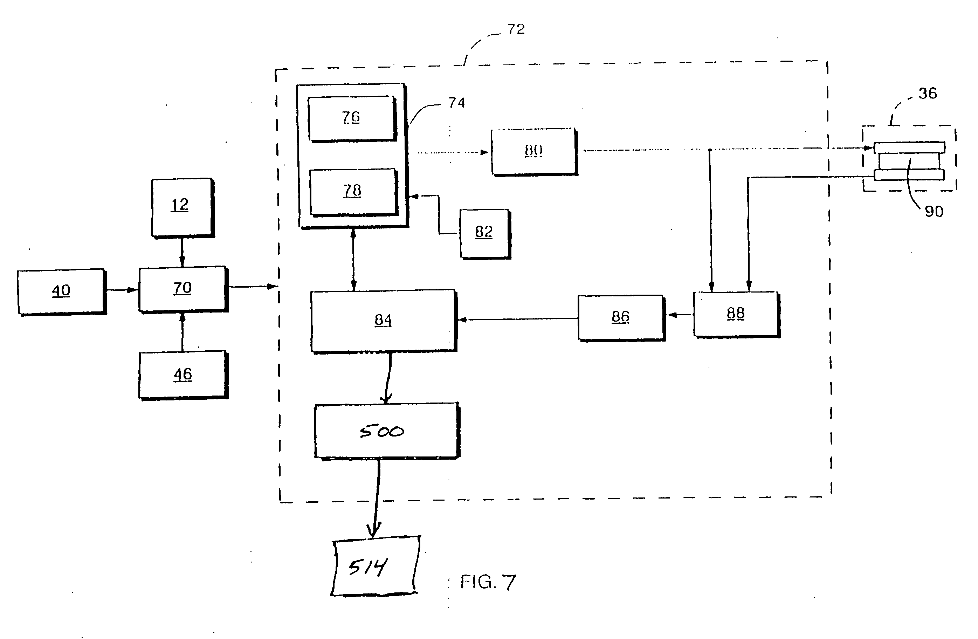

Figure 7 illustrates yet another embodiment of the invention. The Figure 7 embodiment is similar to the Figure 4 embodiment except in the Figure 7 embodiment, clock 500 counts time for the purpose of reminding a patient to use the inhaler. Thus, clock 500 is connected to a tone generator 514.

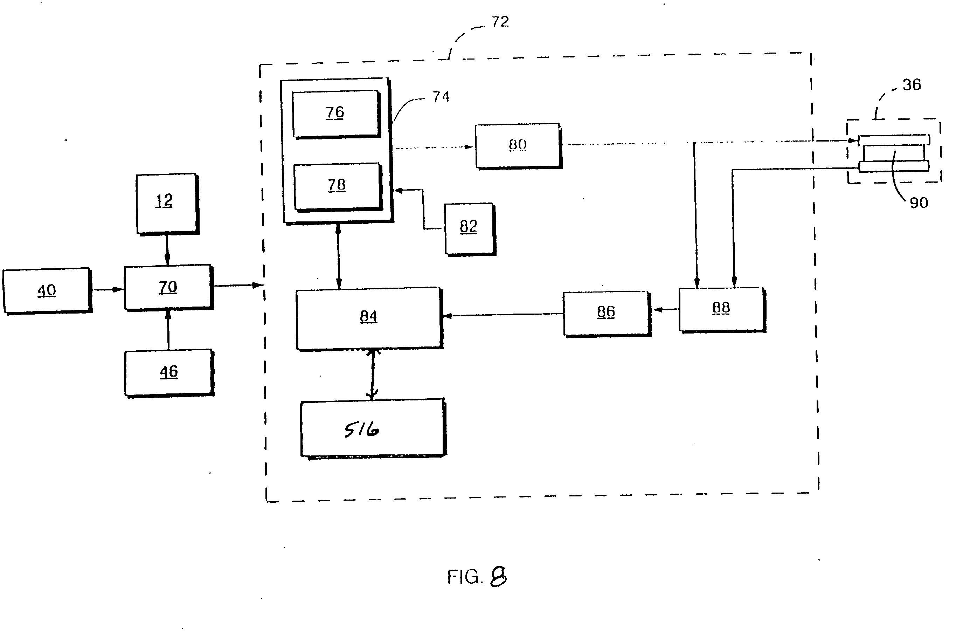

Figure 8 illustrates yet another embodiment of the invention. The Figure 8 embodiment is similar to the Figure 4 embodiment, except that it includes a clock or counter 516 which sends a signal to controller 84 to alter the activation time, i.e. to a shorter or longer period, whereby to alter the quantity of drug delivered, e.g. to increase or decrease dosage over time. Alternatively, clock 516 may be programmed to disable the inhaler once a certain date is passed, i.e. so as to avoid possible use of out-of-date drugs.

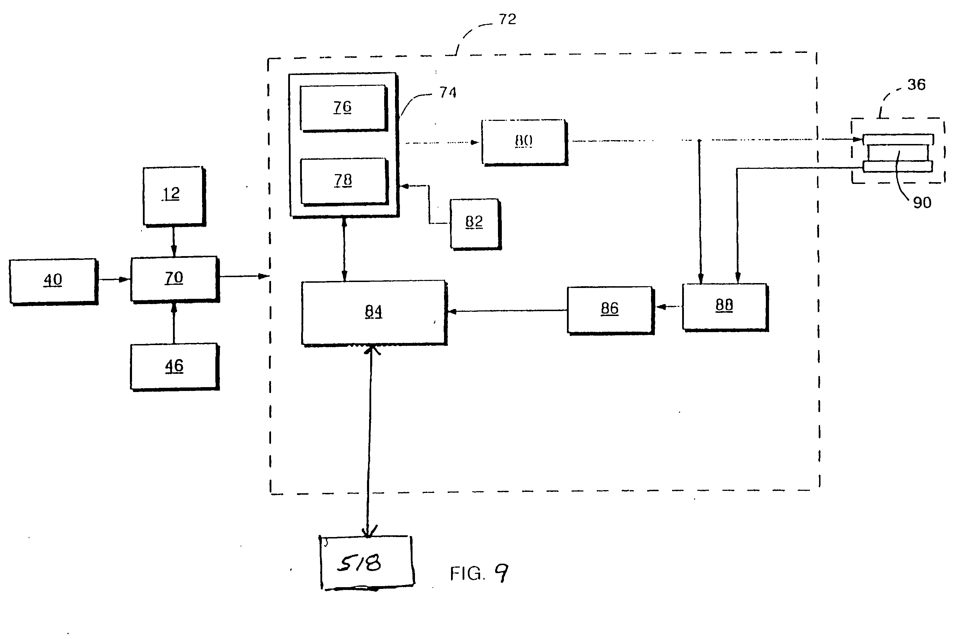

Figure 9 illustrates yet another embodiment of the invention. Figure 9 is similar to Figure 4, except the counter or clock 500 is replaced with a temperature sensor 518. Certain medications are heat sensitive, and may be deactivated, or rendered potentially dangerous if exposed to high temperatures, for example, as might occur if the inhaler is left in an automobile on a sunny day. Temperature monitor 518 will deactivate controller 72 in the event a preset temperature is reached. If desired, monitor 518 also could include a display warning the patient that a preset temperature has been reached.

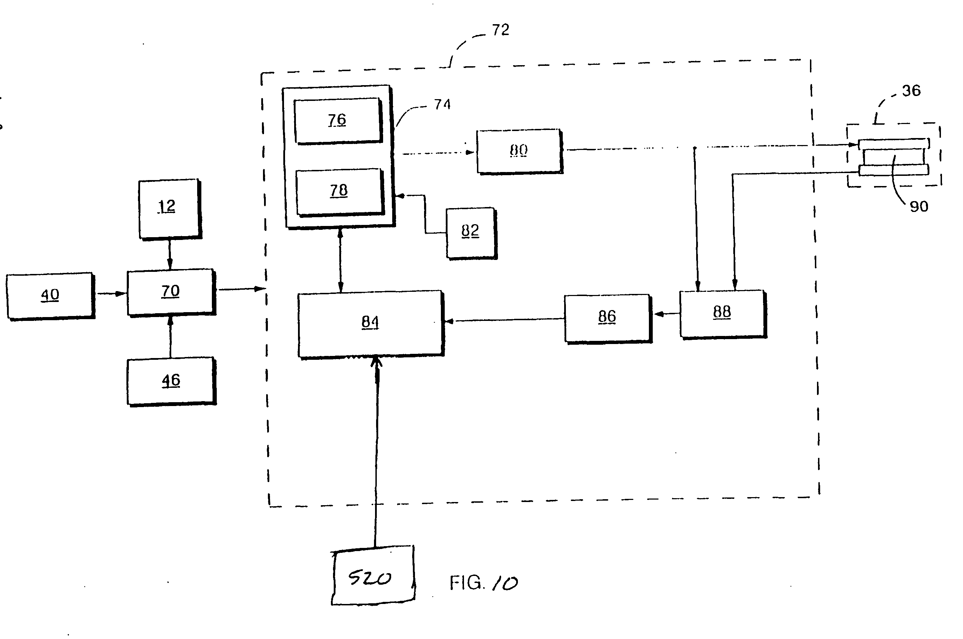

The Figure 10 embodiment is similar to the Figure 9 embodiment except in the Figure 10 embodiment, the temperature sensor is replaced with a "key" such as, for example, a three button keyboard by which the user's pin code must be entered in order to activate the device. This will prevent, for example, use of the inhaler by someone other than the intended patient, and would prevent, for example, controlled or dangerous drugs from being used by children. For ease of use, key 520 may permit the patient (or druggist) to program a specific pin code for the intended user.

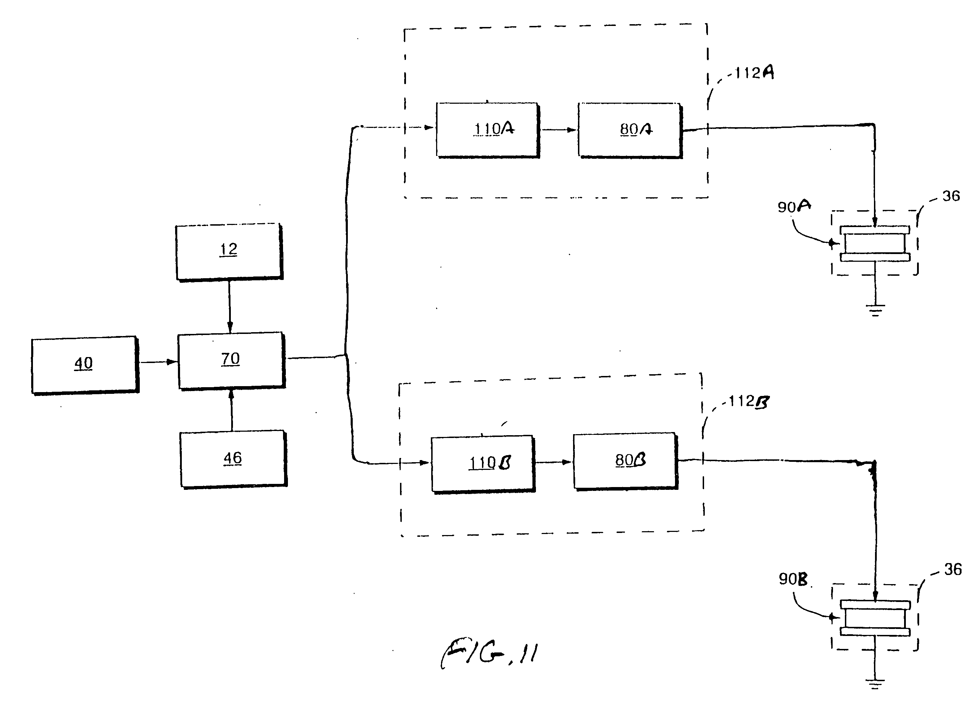

Referring to Figure 11, which illustrates vet another embodiment of the invention, in which two piezoelectric vibrators 90A, 90B, are located side-by-side within the inhaler shell. In this embodiment, piezoelectric elements 90 are designed to vibrate at different amplitudes and frequencies, i.e. so that, for example, two different drugs advantageously may be dispersed simultaneously from the same inhaler, without compromising performance or either drug. This permits delivery of two drugs which, while active together, may not readily be stored together. For example, an asthma inhaler may be provided containing both a bronchodilator, such as albuterol, and a steroid which may require different peizo settings. The Figure 11 embodiment includes a pre-calibrated controller 112 which includes a first and a second pre-calibrated frequency/amplitude control signal generator 110A, 110B, which supplies control signals to pump circuit A and pump circuit B, respectively. Of course, the pre-calibrated controller 112 may be replaced with a pair of feedback controllers similar to that shown in Figure 4.

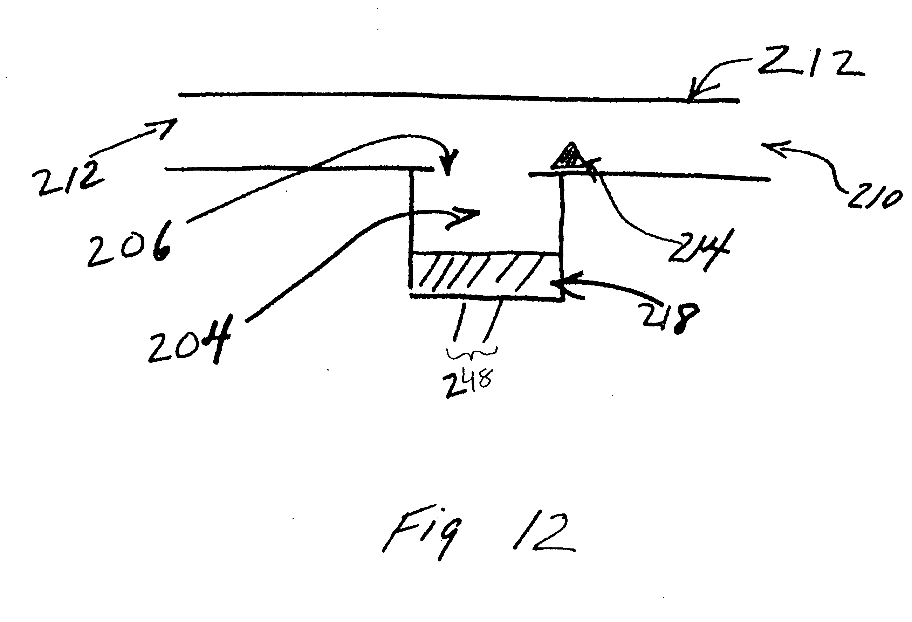

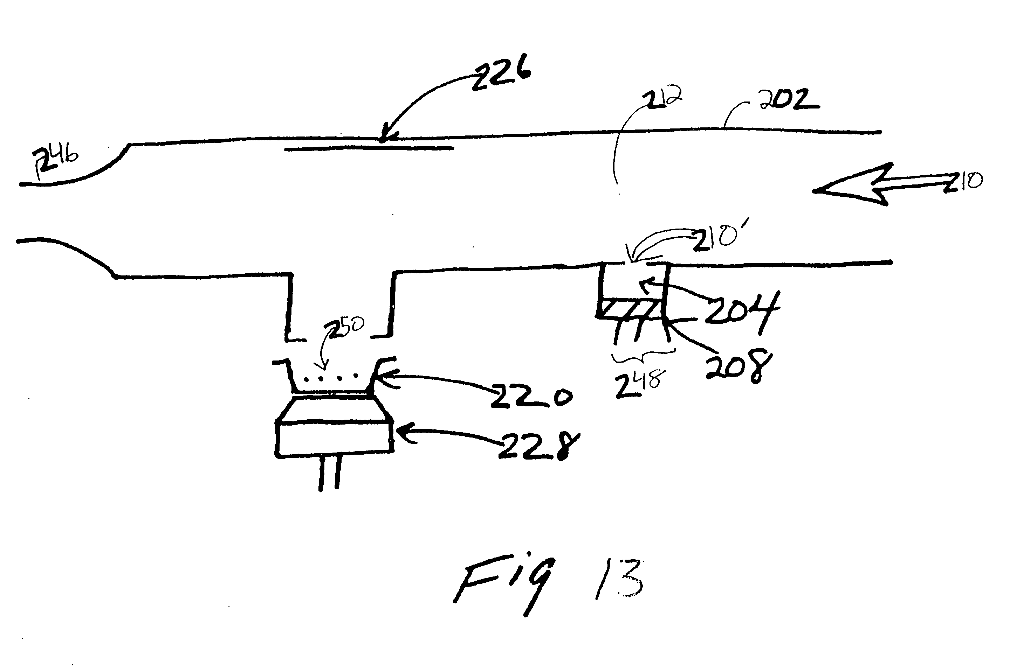

Referring to Figures 12 and 13, a cross-sectional view of an airflow passage 212 of an inhalation device 202 is depicted. It should be noted at the outset that the airflow passage 212 depicted in Figure 12 is a generalized airflow passage of a typical inhalation device, such as those discussed above. However, the present invention is intended to be adapted to any inhalation device, regardless of the particular geometry of the airflow passage. At its most basic level, the present invention operates by providing an air flow sensor 208 to detect air flow turbulence around the sensor 208 (i.e., inspiratory air flow rate of a user of the inhaler) and to control various components of the inhalation device 202, as a function of the amplitude and/or frequency of the detected airflow turbulence, as described below.

As shown in Figure 12, air 110 (or other fluid) enters the airflow passageway 212. typically by the respiratory activity of a patient inhaling on the device 202. As air 210 flows through the passage 212, a portion thereof flows through the opening 206 in the passage 202 into a cavity 204. Placed within the cavity 204 is an air flow sensing device 208. The air flow sensing device 208 is an acoustic sensing device, e.g. a microphone. Microphone 208 is adapted to produce appropriate signals 248 in response to the airflow detected within the cavity 204. The amplitude and frequency of the airflow within the cavity 204 is a function of the airflow rate 210 within the air passage 212 of the device 202. Thus, output signals 248 from the microphone 208 will vary in both frequency and amplitude as a function of air flow rate within the cavity (which is a function of flow rate within the passage 212), and thus, can be used to control various components of the inhaler 202 as a function of frequency and/or amplitude, as described below. Those skilled in the art will appreciate that the shape of the cavity 204 and the size of the opening 206 are chosen in accordance the particular geometry of the air passage 212, the air flow rate 210 through the passage 212. and/or the frequency response and/or sensitivity of the microphone 208; and all such variations are within the scope of the present invention. Preferably, as noted above, the shape of the cavity 204 and the size of the opening 206 are chosen to permit at least a portion of the air within the passage 202 to enter the cavity 204 with sufficient amplitude to induce a response from the microphone 208.

Referring now to Figure 13, an expanded cross-sectional view of an embodiment of the air flow sensor (described with reference to Figure 12, above) in a dry powder inhaler, such as disclosed in U.S. Patent 5,694,920. Depicted in Figure 13 are the components of a typically dry powder inhaler 202. A mouthpiece 246 is provided for a user (i.e., patient) to inhale on the device 202. A high-frequency vibratory mechanism 228 (e.g., piezoelectric element, ultrasonic acoustic transducer, or other electro/mechanical vibratory mechanism, etc.) is provided to vibrate a container 220 (e.g., blister capsule) of dry powdered medicament 250 to suspend particles of the medicament into the air passage 212. To further aid the suspension of particles, an electrostatic potential plate 226 can be provided to draw particles of a certain charge (i.e., a charge opposite to that of the electrostatic plate 226) into the air stream 210. In this embodiment, a portion 210' of the air 210 drawn into the air passage 212 is induced into the cavity 204, to be detected by the microphone element 208. Upon detection of airflow, the microphone element produces output signals 248 proportional in amplitude and frequency of the air flow rate within the air passage 212. The output signals 248 are used to control either the high-frequency vibrator 228 and/or the electrostatic plate 226, or other components of the inhaler, as described below.

Figure 14 is a block diagram representation of the acoustic control system of the present invention for a dry powder inhaler. As described above, the microphone element 208 produces signals 248 in response to detected airflow 210'. These signals are processed by an amplitude/frequency processor 230 to condition the signals 248 and to determine the amplitude and/or frequency of the output signals 248. The amplitude/frequency processor produces output signals 248' to control the high-frequency vibrator and/or electrostatic plate. To that end, output signals 248' arc input into a comparator circuit 240 and/or 232 and compared with a reference threshold signal 242 and or 234, respectively.

It should be understood that signals 248 and 248' are indicative of the airflow rate 210, described above. The present invention is intended to be controllable as a function of frequency and/or amplitude of signals 248, thus, amplitude/frequency processor can be adapted to condition the signals 248 in terms of amplitude or frequency are both. High frequency vibrator threshold 242 produces a signal 252 which represents the minimum voltage and/or frequency required to activate the high frequency vibrator controller 244 (which, in turn, activates the high frequency vibrator 226). Comparator 240 compares signal 252 with signal 248' and if the signals have equal amplitude and/or frequency (within some predetermined error margin), comparator activates the high frequency vibrator controller 244, which activates and directly controls the high frequency vibrator 226. Similarly, electrostatic plate deflector controller 236 is activated by an equal match of signals 248' and 254 by the comparator 232. Electrostatic plate detector threshold 234 produces signal 254 which represents the minimum voltage and or frequency required to activate the electrostatic plate 226.

Inspiratory capacity processor 238 is provided to compute the peak inspiratory flow 210 (represented by signals 248 and 248') of the patient. Although not shown in the drawings, this information can be used to adjust the threshold signals of the high frequency vibrator threshold 242 and/or electrostatic plate detector threshold 234. Of course, to accomplish this, the high frequency vibrator threshold 242 and / or electrostatic plate detector threshold 234 must be programmable, as is known in the art. In this way, the microphone 208 can be programmed to trigger the various components of the inhaler to adjust for varying inspiration flow rates from patient-to-patient or individually. Thus, for example, the inspirator control scheme of the present invention can be self-adjusting to account for a patient's decrease in inspiratory flow rate caused by, for example, decreased lung capacity. Alternatively, the processor 238 can be modified to sequentially turn on the various components herein described (e.g., vibrator, electrostatic plate, etc.) at optimal inhalation times (e.g., peak inhalation effort). Thus, for example, the processor 238 can be modified to activate the vibrator at a time just prior to the user's peak inhalation effort, then to activate the electrostatic plate subsequently, thereby inducing the medicament into the airstream at a time that produces optimal respiratory absorption of the medicament. Moreover, processor 238 can be adapted with appropriate memory to track a patient's inspiratory flow rate which can be used to adjust the powdered medicament 250 to achieve maximum medication benefit.

Many modifications, alternatives and equivalents are possible. For example, Processor 230, threshold signal generators 234 and 242, comparators 242 and 232 and can be any known digital (e.g., microprocessor) or analog circuitry and/or associated software to accomplish the functionality described herein. Although the various components described in Figure 14 have been described in a modular fashion, those skilled in the art will recognize that each of these components can be discrete off-the-shelf or custom components, or can be included in a single, unified system.

The present can be modified by permitting the microphone signals 248 and 248' to directly control activation of the high frequency vibrator 228 and/or electrostatic plate 226, thereby bypassing the comparators 240 and/or 232. In this way, microphone 208 can be adapted to activate these components in a binary fashion that is not dependent upon How rate. Also, it will be understood to those skilled in the art that the thresholding circuits 242 and 234, the amplitude/frequency processor 230 and the inspiratory capacitor processor 238 can be adapted to permit user (patient) control and user-definable presets (i.e., minimum flow rate for activation, etc).

In addition, comparators 240 and 232 can be adapted to permit generation of activation signals based differing signal strengths and/or frequency. Thus, for example, the high frequency vibrator can be adapted to activate only when a signal frequency of 1Khz is achieved, while the electrostatic plate will only activate when a signal strength of 35mV. is obtained.

Other modifications are also possible. For example, the microphone 208 can be positioned directly on the inner wall of the airflow passage 212 of the device 202. instead of within the cavity 204. Also, as shown in Figure 12, a turbulence generator 214 can be provided to generator air turbulence within the air passage 212. This modification, for example, can be used in an inhalation device that would otherwise not permit a portion 210' of the air 210 to enter the cavity 204. In addition, instead of a microphone 208, the acoustic element can be any known fluid pressure transducer (e.g., air pressure transducer) that will output appropriate signals as a function of fluid pressure (amplitude) and/or frequency. Accordingly, the present invention can be appropriately modified to operate in any fluid medium (other than air), to provide automatic acoustic control.

Still other modifications are possible. For example, although not shown in the drawings, the present invention can be provided with a timer that is controlled by signals 248'. The timer can be appropriately modified to control a schedule of when the device may be activated, to avoid, for example, an overdose. Thus, for example, the timer may be modified to only permit activation of the components of the device at certain times of the day. Moreover, the timer may be appropriately modified to permit downloading of data related to usage (e.g., time of day used, dosage of medicament, inhalation effort, etc.). This data can be particularly relevant for clinical trials where it is important to track the recommended dosage and times of medication. Of course, the previous description could be accomplished with a counter, or the like, that simply counts the amount of times that the device has been used.

Although the present invention has been directed to an acoustic control scheme for a dry powder inhaler 202, the present invention is not so limited. On the contrary, the present invention is intended to be adapted for any inhalation device that would require a control mechanism (such as described herein) based breath (inhalation) detection. For example, an anesthetic device could be modified with the breath sensor and controller as provided herein to monitor and control the amount of anesthetic a patient receives. Additionally, the acoustic sensing element can be used to measure peak inspiratory and / or expiratory flow of a particular patient, and record this information for downloading and analvsis.

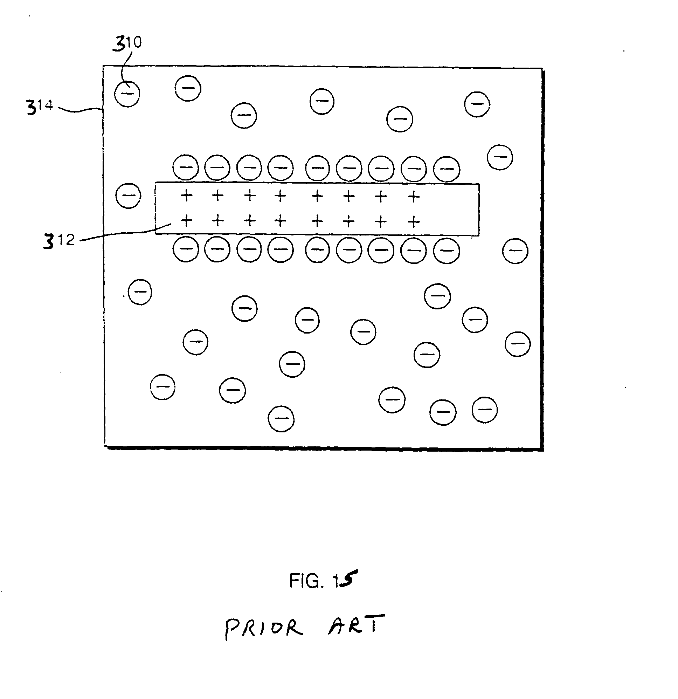

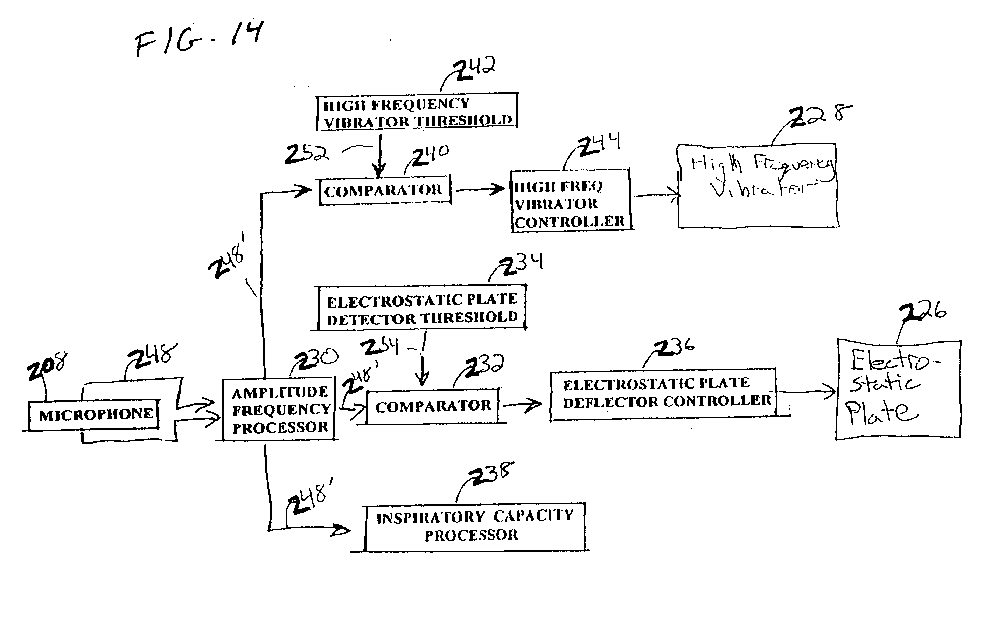

Referring to Figure 15 there is illustrated a chamber 314 containing aerosolized dry powder particles of a pharmaceutical or drug 310. These particles 310 are suspended in air and carry a charge, for example a negative charge. Also in the chamber is a support surface 312 having a charge opposite to that on the particles. The support surface 312 will attract a number of charged particles 310 sufficient to neutralize the charge on the surface of the support 312. This support surface is one that can hold a discrete electrical charge on its surface, such as insulating material, e.g. plastic or a semiconductor material, such as selenium, used in the photocopy industry.

The actual amount of pharmaceutical or drug powder transferred to the carrier sheet is a function of the mass-to-charge ratio of the powdered particles. If one assumes surface charge saturation, the amount of charge carried by the particles is directly related to the surface area. For spheriodal particles, the charge varies as the square of the radius and the mass varies as the cube. Thus, the amount of charged particles picked up by a given portion of the surface of the charge carrier will be a function the total charge on the carrier. Thus, with a given surface charge density on the carrier, the amount of pharmaceutical or drug powder picked up is directly proportional to the charged area. Thus, for doubling the amount of pharmaceutical or drug powder to be picked up, and thus the dose amount, the area on which charge is placed can be doubled. This can be used as a basic method to control the amount of powder to be picked by the carrier. Thus, for any particular powder or particle size distribution of powder, the exact area and amount of charge needed can be experimentally determined.

Still other modifications and variations of the invention described herein may be made and are intended to come within the scope of the appended claims.

## Claims

1. An inhalation device (202) for delivering powdered medication from a medication storage container (220) to a patient, comprising:
a passage (212) through which said powdered medication is delivered from said medication storage container,
a controller disposed in said passage, said controller comprising an acoustic element (208) adapted to produce signals (248) representative of frequency and/or amplitude of said fluid flow turbulence, or a fluid pressure transducer adapted to produce signals as a function of fluid pressure amplitude and/or frequency, a high frequency vibrator (228) to vibrate and deaggregate powdered medication to induce said medication into said fluid and, as an option, an electrostatic plate (226) to attract particles towards said electrostatic plate and into said fluid, the activation/deactivation of said high frequency vibrator (228) and of said electrostatic plate being controlled by the output signals (248) of said acoustic element (208) or of said fluid pressure transducer.

2. An inhalation device according to claim 1, wherein said acoustic element (208) is a microphone.

3. An inhalation device according to claim 1 or claim 2, wherein said controller further comprises an inspiratory capacity processor (238) for processing data related to a fluid flow rate around said acoustic element or said fluid pressure transducer and for using said data to further control said activation/deactivation, said data preferably including minimum and maximum flow rate which is indicative of inspiratory and/or expiratory effort of a user of said inhalation device.

4. An inhalation device according to any of claims 1 to 3, wherein said controller further comprises a timer for controlling time/frequency activation of said inhalation device.

## Patentansprüche

1. Inhalationsvorrichtung (202) zur Zufuhr eines pulverförmigen Medikaments von einem Medikamentenspeicherbehälter (220) zu einem Patienten, umfassend:
eine Durchgangsleitung (212), durch die das pulverförmige Medikament aus dem Medikamentenspeicherbehälter zugeführt wird,
eine in der Durchgangsleitung angeordnete Steuereinrichtung, welche Steuereinrichtung ein akustisches Element (208) umfasst, das dazu ausgelegt ist, Signale (248) zu erzeugen, die für die Frequenz und/oder Amplitude der Fluidströmungsturbulenz repräsentativ sind, oder einen Fluiddruckwandler umfasst, der dazu ausgelegt ist, Signale als Funktion einer Fluiddruckamplitude und/oder -frequenz zu erzeugen, ferner eine Hochfrequenzschwingungseinrichtung (228), um das pulverförmige Medikament in Schwingung zu versetzen und zu desaggregieren, um das Medikament in das Fluid einzubringen, sowie als Option eine elektrostatische Platte (226), um Partikel zur elektrostatischen Platte hin und in das Fluid anzuziehen, wobei die Aktivierung/Deaktivierung der Hochfrequenzschwingungseinrichtung (228) und der elektrostatischen Platte durch die Ausgangssignale (248) des akustischen Elements (208) oder des Fluiddruckwandlers gesteuert/geregelt wird.

2. Inhalationsvorrichtung nach Anspruch 1, wobei das akustische Element (208) ein Mikrofon ist.

3. Inhalationsvorrichtung nach Anspruch 1 oder Anspruch 2, wobei die Steuereinrichtung ferner einen Atmungskapazitätsprozessor (238) umfasst, um Daten betreffend eine Fluidströmungsrate um das akustische Element oder den Fluiddruckwandler herum zu verarbeiten, und um die Daten zur weiteren Steuerung/Regelung der Aktivierung/Deaktivierung zu verwenden, wobei die Daten vorzugsweise eine minimale und maximale Strömungsrate enthalten, die die Einatmungs- und/oder Ausatmungsanstrengung eines Benutzers der Inhalationsvorrichtung angibt.

4. Inhalationsvorrichtung nach einem der Ansprüche 1 bis 3, wobei die Steuereinrichtung ferner einen Zeitgeber zum Steuern/Regeln der Zeit-/Frequenzaktivierung der Inhalationsvorrichtung umfasst.

## Revendications

1. Dispositif d'inhalation (202) destiné à délivrer des médicaments en poudre à partir d'un récipient de stockage de médicaments (220) à un patient, comprenant :
un passage (212) à travers lequel ledit médicament en poudre est délivré à partir dudit récipient de stockage de médicaments,
un appareil de contrôle disposé dans ledit passage, ledit appareil de contrôle comprenant un élément acoustique (208) adapté pour produire des signaux (248) représentatifs de la fréquence et/ou de l'amplitude de ladite turbulence d'écoulement de fluide ou un transducteur de pression de fluide adapté à la production de signaux en fonction de l'amplitude et/ou de la fréquence de la pression de fluide, un vibrateur à haute fréquence (228) pour faire vibrer et désagréger le médicament en poudre afin d'induire ledit médicament dans ledit fluide et, en option, une plaque électrostatique (226) pour attirer des particules vers ladite plaque électrostatique et dans ledit fluide, l'activation/ la désactivation dudit vibrateur à haute fréquence (228) et de ladite plaque électrostatique étant commandées par les signaux de sortie (248) dudit élément acoustique (208) ou dudit transducteur de pression de fluide.

2. Dispositif d'inhalation selon la revendication 1, dans lequel ledit élément acoustique (208) est un microphone.

3. Dispositif d'inhalation selon la revendication 1 ou la revendication 2, dans lequel ledit appareil de contrôle comprend en outre un processeur de capacité inspiratoire (238) destiné à traiter des données relatives à un débit de fluide autour dudit élément acoustique ou dudit transducteur de pression de fluide et destiné à utiliser lesdites données pour mieux commander ladite activation/ désactivation, lesdites données comprenant de préférence un débit minimum et un débit maximum qui indiquent l'effort d'inspiration et/ou d'expiration d'un utilisateur dudit dispositif d'inhalation.

4. Dispositif d'inhalation selon l'une quelconque des revendications 1 à 3, dans lequel ledit appareil de contrôle comprend en outre un minuteur destiné à commander l'activation temps/fréquence dudit dispositif d'inhalation.
